# EUROPEAN PATENT APPLICATION

(11) **EP 2 465 841 A1**
(43) Date of publication of application: **20.06.2012**
(21) Application number: 10191856.3
(22) Date of filing: 19.11.2010
(51) Int. Cl.: C07C 67/08, C07C 69/63, C07C 69/24, C07C 69/78, C07C 51/363, C07C 53/16, C07C 53/19

(54) **Preparation of esters of mono- or dicarboxylic non-aromatic and aromatic acids**

(71) Applicant: Lonza Ltd, 4052 Basel (CH)
(72) Inventor: Litzmann, Oliver, 12203 Berlin (DE); Repke, Jens-Uwe Prof., 12621 Berlin (DE); Hanselmann, Paul Dr., 3902 Brig-Glis (CH); Heyl, Andreas Dr., 3912 Termen (CH)
(74) Representative: Winter, Brandl, Fürniss, Hübner, Röss, Kaiser, Polte - Partnerschaft

(57) **Abstract**

The present invention relates to a method for preparing a carboxylic acid ester, wherein a carboxylic acid and/or a salt thereof is reacted with an alcohol in the presence of hydrogen chloride (HCl) and water to form the carboxylic acid ester, wherein the reaction is carried out using a starting reaction system wherein the molar ratio of HCl to carboxylic acid is at least 0.075 : 1 and the molar ratio of water to carboxylic acid is at least 0.25 : 1.

## Description

The present invention relates to a method for the preparation of carboxylic acid esters from carboxylic acids and alcohols in the presence of hydrogen chloride.

Mono- and dicarboxylic acid esters are valuable chemical products in industry where they are used, for example, as chemical building blocks for polymers, as plasticizers for plastics, as fuel replacements for combustion engines, as softeners for resists and photoresists, or as intermediates in organic syntheses.

The synthesis of carboxylic acid esters from carboxylic acids and alcohols is well known in the art.

According to conventional methods, esters are formed by reacting a carboxylic acid and an alcohol in the absence of water and in the presence of catalytic amounts of acid. To shift the equilibrium of the reaction to the carboxylic acid ester, the water formed during the condensation reaction is typically removed by azeotropic distillation together with the formed carboxylic acid ester or it is bound by concentrated sulphuric acid. In an alternative method, the extractive esterification, the carboxylic acid ester formed in the reaction is extracted into a solvent which is poorly miscible with the formed water (Organikum, 23. Edition, p.481, 2009, Wiley-VCH, Weinheim, Germany).

DE-A-195 39 962 and DE-A-37 37 759 describe methods for the continuous production of esters of chloroacetic acid in the presence of a catalyst in a homogenous reaction system. Both methods require the continuous removal of ester and water to achieve satisfying product yields.

It would therefore be desirable to have a simple method with high product yields without the need of water-free reaction conditions which are elaborate to maintain and to control.

The object of the present invention, thus, is the provision of a simple and effective method for the synthesis of carboxylic acid esters which results in high product yields.

This object has been achieved by the method of the invention, wherein a carboxylic acid and/or a salt thereof is reacted with an alcohol in the presence of hydrogen chloride (HCl) and water to form the carboxylic acid ester, wherein the reaction is carried out using a starting reaction system wherein the molar ratio of HCl to carboxylic acid is at least 0.075 : 1 and the molar ratio of water to carboxylic acid is at least 0.25 : 1.

In has surprisingly been found that the use of HCl in amounts exceeding conventional catalytic amounts in the continuous presence of water allows the production of carboxylic acid esters in high yields even with sensitive unsaturated carboxylic acids or dicarboxylic acids. Unexpectedly, the presence of water in the starting reaction mixture and the formed reaction water have no adverse effect on the product yield, and water and ester product need not be removed during the reaction.

The molar ratio of HCl to carboxylic acid (any amount of HCl calculated on the basis of 100% HCl) is preferably at least 0.1 : 1, more preferably at least 0.2 : 1. The upper limit for HCl is not critical, but typically the molar ratio of HCl to carboxylic acid does not exceed 2 : 1. Usually, the molar ratio of HCl to carboxylic acid is the range of from 0.1 : 1 to 2 : 1, in particular of from 0.2 : 1 to 1.5 : 1, and most preferably in the range of from 0.2 : 1 to 0.4 : 1, for example about 0.3 : 1. The hydrogen chloride may be fed to and can be present in the reaction system in gaseous form and/or in the form of an aqueous solution, i.e., hydrochloric acid. The aqueous solution may be a concentrated hydrochloric acid solution (for example an aqueous solution containing 36% by weight of HCl) or a diluted hydrochloric acid solution. Preferably, hydrogen chloride is added in the form of an aqueous solution.

Water may be added separately to the reaction system but conveniently is added together with the HCl in the form of an aqueous solution of HCl as described above. The amount of water in the starting reaction system is not critical as long as the molar ratio of water to carboxylic acid is at least 0.25 : 1. Preferably, the molar ratio of water to carboxylic acid is at least 0.35 : 1 and more preferably is at least 0.7 : 1. As described above, HCl is usually added in the form of an aqueous solution and, therefore, the amount of water will typically increase with increasing amounts of HCl. For example, if the molar ratio of HCl to carboxylic acid is 0.1 : 1 or more, the molar ratio of water to carboxylic acid is at least 0.35 : 1. For practical reasons, water may be present in an amount which is about equimolar to that of the carboxylic acid. Typically, the molar ratio of water to carboxylic acid does not exceed 3 : 1 and preferably does not exceed 2 : 1.

The carboxylic acid used in the method of the present invention may be present in the reaction system in the form of the free acid and/or in the form of a salt thereof, in particular in the form of an alkali metal salt, such as a lithium, sodium or potassium salt.

The carboxylic acid used in the invention preferably is a linear or branched saturated or unsaturated aliphatic C₂₋₂₀ monocarboxylic or dicarboxylic acid or a mono- or bicyclic aromatic mono- or dicarboxylic acid. Aliphatic as well as aromatic acids may optionally be halogenated with one or more, preferably one to three halogen atoms. Suitable halogen atoms are selected from the group consisting of fluorine, chlorine, bromine or iodine. Preferably, the halogen atom is a chlorine atom.

In a preferred embodiment, the carboxylic acid is a linear or branched saturated or unsaturated aliphatic C₂₋₆ mono- or dicarboxylic acid, said acid being optionally halogenated with one or more halogen atoms. Preferably, these acids are used in the form of the free acid.

Examples of preferred aliphatic C₂₋₆ monocarboxylic acids useful in the present invention are linear or branched saturated monocarboxylic acids such as acetic acid, propionic acid, 2,2-dimethyl propionic acid, butyric acid, isobutyric acid, pentanoic acid, 2-methyl butanoic acid, 3-methyl butanoic acid, 2,2-dimethyl butanoic acid, 2-methylpentanoic acid, 3-methylpentanoic acid, 4-methylpentanoic acid, hexanoic acid, 2-ethylbutanoic acid or 3-ethylbutanoic acid, which may optionally be halogenated with one or more halogen atoms as defined above. Preferred halogenated C₂₋₆ monocarboxylic acids are linear or branched saturated monocarboxylic acids carrying one or two halogen atoms, preferably one halogen atom. Particularly preferred are halogenated, in particular chlorinated C₂₋₄ monocarboxylic acids having one to three chlorine atoms, preferably one chlorine atom, such as monochloroacetic acid (chloroacetic acid), dichloroacetic acid, trichloroacetic acid, 2-chloropropionic acid, 3-chloropropionic acid, 3,3-dichloropropionic acid or 3,3,3-trichloropropionic acid Preferred carboxylic acids are acetic acid, chloroacetic acid, dichloroacetic acid, 2-chloropropionic acid and 3-chloropropionic acid.

Preferred linear or branched saturated or unsaturated aliphatic dicarboxylic acids are C₂₋₆ dicarboxylic acids such as oxalic acid, malonic acid, adipic acid and sebacic acid, which may optionally be halogenated with one or more, preferably one or two halogen atoms, preferably chlorine atoms.

In a further preferred embodiment, the carboxylic acid of the present invention is a linear or branched saturated or unsaturated aliphatic C₁₂₋₂₀ mono- or dicarboxylic acid, said acid being optionally halogenated with one or more halogen atoms. More preferably, the carboxylic acid is an optionally halogenated linear saturated or unsaturated aliphatic C₁₂₋₂₀ monocarboxylic acid. Most preferably, the C₁₂₋₂₀ monocarboxylic acid is a saturated or mono- or polyunsaturated acid such as lauric acid, myristic acid, palmitic acid, heptadecanoic acid, stearic acid, arachidic acid, palmitoleic acid, oleic acid, elaidic acid, α- or β-linolenic acid, linoleic acid, punicic acid and arachidonic acid.

In a further preferred embodiment, the carboxylic acid of the present invention is a mono- or bicyclic aromatic monocarboxylic or dicarboxylic acid, said acid being optionally halogenated with one or more, in particular one to three halogen atoms, preferably chlorine atoms.

Preferably, the aromatic monocarboxylic acid is a non-halogenated or halogenated, in particular chlorinated, benzoic acid or naphthoic acid. Examples of non-halogenated and halogenated monocarboxylic acids useful in the present invention are benzoic acid, 2-chlorobenzoic acid, 3-chlorobenzoic acid, 4-chlorobenzoic acid, 1-naphthoic acid, 2-naphthoic acid, 2-chloro-1-naphthoic acid, 3-chloro-1-naphthoic acid, 4-chloro-1-naphthoic acid, 1-chloro-2-naphthoic acid, 3-chloro-2-naphthoic acid, 4-chloro-2-naphthoic acid, 5-chloro-1-naphthoic acid, 8-chloro-1-naphthoic acid, 5-chloro-2-naphthoic acid, 6-chloro-2-naphthoic acid or 7-chloro-2-naphthoic acid. Benzoic acid and halogenated, in particular chlorinated derivatives thereof as described above are most preferred.

Examples of aromatic dicarboxylic acids useful in the present invention are phthalic acid, isophthalic acid, terephthalic acid, naphthalene 2,6-dicarboxylic acid, naphthalene 1,8-dicarboxylic acid and naphthalene 2,8-dicarboxylic acid. Phthalic acid, isophthalic acid and terephthalic acid are most preferred.

The alcohol used in the method of the present invention preferably is a linear or branched saturated or unsaturated aliphatic C₁₋₈ alcohol, preferably a monovalent alcohol. Preferably the alcohol is a saturated alcohol. C₁₋₈ alcohols useful in the present invention may be for example methanol, ethanol, propanol, isopropyl alcohol, *n*-butanol, sec-butanol, tert-butanol, pentanols such an n-pentanol, hexanols such as *n*-hexanol, heptanols such as *n*-heptanol, and octanols such as *n*-octanol. C₁₋₆ alcohols are particularly preferred, with C₂₋₄ alcohols such as ethanol, propanol, isopropyl alcohol, *n*-butanol, sec-butanol and tert-butanol being more preferred. Ethanol, propanol and isopropyl alcohol are particularly preferred, with ethanol being most preferred.

The alcohol may be present in the starting reaction system in a molar ratio of alcohol to carboxylic acid of at least 0.8 : 1, preferably a molar ratio in a range of from 0.8 : 1 to 4 : 1, more preferably of from 1 : 1 to 1.5 : 1.

It has also been found that the presence of hydrogen chloride in the amounts according to the invention favours the formation of a biphasic liquid-liquid system with an aqueous and an organic phase containing a high content of ester product even if both the carboxylic acid and the alcohol used in the reaction are water miscible. According to a preferred embodiment of the invention, therefore, the reaction is carried out under conditions adjusted so that a biphasic liquid-liquid system is present at least after completion of the reaction when the ester has formed, which also facilitates the isolation of the ester product. Conditions can be easily adjusted by properly selecting the relative amounts of alcohol and water in the starting reaction system at a given amount of HCl.

Preferably, the relative amounts of alcohol and water in the starting reaction system are selected so that a biphasic liquid-liquid system is present at a temperature of 25°C and a pressure of 1 bar at least after completion of the reaction. More preferably, the relative amounts are selected so that the biphasic liquid-liquid system forms in the course of the reaction under the reaction conditions, for example if higher or lower temperatures and pressures are applied during the reaction, for example temperatures and pressures as defined below.

Preferably, thus, the method of the invention is carried out using a starting reaction system wherein the molar ratio of alcohol to carboxylic acid in the starting reaction mixture is in the range of from 0.8 : 1 to 4 : 1, preferably of from 1 : 1 to 1.5, the molar ratio of HCl to carboxylic acid is at least 0.1 : 1, preferably at least 0.2 : 1, and the molar ratio of water to carboxylic acid is at least 0.35 : 1, preferably at least 0.7 : 1.

Under the above conditions, the reaction is essentially carried out in said biphasic system (i.e., within the miscibility gap). It is assumed that the reaction takes place either in the aqueous phase or at the liquid-liquid boundary with the organic phase which is present either due to the presence of water-immiscible acids or alcohols or due to the forming ester product. Thus, with the method according to the present invention higher yields may be achieved than with the prior art methods using only catalytic amounts of hydrogen chloride.

The relative molar amounts of carboxylic acid, alcohol, water and HCl at which a biphasic reaction system will form under reaction conditions and even in the presence of water miscible alcohols can be determined by simple experiments (see Example 13). In particular, at a given molar ratio of HCl to carboxylic acid, the amounts of water and alcohol can be adjusted to form the biphasic system. Generally, the miscibility gap will disappear with increasing amounts of alcohol, in particular lower alcohols such as ethanol, which act as a solubilizer. Preferably, the alcohol will be present in a molar excess with respect to the carboxylic acid as this shifts the equilibrium to the ester product, but only in such amounts that the biphasic system is maintained. In general, the molar ratio of alcohol to carboxylic acid will not exceed 4 : 1. Preferably, the molar ratio of carboxylic acid to alcohol will be in the range of from 1 : 1 to 1 : 1.5, for example about 1 : 1.3.

While the starting reaction system may consist of carboxylic acid, alcohol, water and HCl alone, the starting reaction system may also comprise an inert water-immiscible organic solvent in order to dissolve solid carboxylic acids and/or to assist the formation of a biphasic liquid-liquid system. This may be advantageous in cases where no clear liquid-liquid boundaries or emulsions are formed as may be the case with alcohols such as butanol. The inert solvent will preferably be present in the reaction system in a molar ratio of solvent to carboxylic acid of from 0.1 : 1 to 4 : 1.

Examples for water-immiscible organic solvents useful in the present invention are aliphatic, cycloaliphatic and aromatic hydrocarbons with 5 to 10 carbon atoms, in particular C₅₋₈ alkanes such as *n*-pentane, *n*-hexane, *n*-heptane, *n*-octane and *iso*octane, C₅₋₈ cycloalkanes such as cyclopentane, cyclohexane, cycloheptane and cyclooctane, aromatic hydrocarbons such as benzene, toluene and xylenes, halogenated hydrocarbons such as dichloromethane, chloroform, tetrachloromethane, and mixtures thereof.

The method of the present invention can be carried out by mixing carboxylic acid, alcohol, HCl, water and, optionally, organic solvent in a reaction vessel and heating the obtained mixture to the desired reaction temperature. The reaction mixture is stirred at the reaction temperature until the reaction is complete. Typically, the reaction time is not more than about 8 h. Typically, the reaction has reached equilibrium after about 0.5 h and thus allows high space-time yields.

The method of the invention can be carried out over a wide temperature range but typically is carried out at a reaction temperature of from 20 to 70 °C, more preferably of from 30 to 60 °C. Likewise, pressure is not a critical feature, but for convenience the reaction is carried out at a pressure in the range of from about 1 bar (ambient pressure) to 5 bar, preferably at a pressure of about 1 bar. If the reaction is carried out at a pressure of more than 1 bar, reaction temperatures may also be higher than 70 °C.

The ester obtained by the above reaction may be isolated according to known methods, for example by distillation or by separating the aqueous and the organic layers, washing the organic layer with water and removing residual carboxylic acid, alcohol, and, if present, organic solvent.

In case the carboxylic acid to be used for esterification in the present method is a chlorinated saturated, in particular linear C₂₋₆ monocarboxylic acid, for example a mono-chlorinated carboxylic acid such as monochloroacetic acid, 2-chloropropionic acid or 3-chloropropionic acid, said chlorinated carboxylic acid may be prepared before the esterification step in a further reaction step wherein the corresponding non-chlorinated carboxylic acid is reacted with chlorine according to methods known in the art to obtain a mixture containing said chlorinated carboxylic acid and hydrogen chloride. The starting reaction system used in the method of the invention may then be obtained by adding the alcohol, and, optionally, water and HCl in the required amounts to said reaction mixture without the need of further purification and/or separation of the reaction products of the chlorination reaction. As HCl is formed in the chlorination reaction, it is usually not necessary to add further HCl to the reaction system. Typically, the molar ratio of HCl to carboxylic acid is in the range of from 0.8 : 1 to 1.2 : 1. Preferably, as described above, alcohol and water are added in amounts selected to provide a starting reaction system which finally results in the formation of a biphasic reaction system. As a practical example, if a mono-chlorinated carboxylic acid is to be obtained, preferably 1 eq. of carboxylic acid is reacted with 1 eq. of gaseous chlorine to obtain 1 eq. of mono-chlorinated carboxylic acid and 1 eq. of HCl, and after completion of the reaction, preferably 1.3 eq. of alcohol and 1 eq. of water are added in order to obtain the starting reaction system used in the present invention. The esterification reaction may then be carried out in the same reaction vessel as the chlorination reaction.

The invention is further illustrated by the following examples which do not limit the scope of the invention.

### Examples

Working examples were performed at various HCl concentrations with different acids and alcohol. If not indicated otherwise, the respective acid was mixed with the alcohol in the reactor vessel in the indicated molar ratio and the mixture was stirred and heated to a reaction temperature of 60 °C. HCl and water were fed together in the form of a concentrated aqueous solution of HCl (fuming HCl; 36% by weight of HCl in water) to obtain a molar ratio of HCl to carboxylic acid in a range of from 0.1 to 0.33 (calculated on 100% HCl, i.e., gaseous HCl), respectively. The mixture was stirred and thermostated at the reaction temperature for the indicated period of time, and then liquid samples were taken from the biphasic system and analyzed using Gas Chromatography with Flame Ionization Detector (GC FID) to determine the yield in ester with reference to the starting amount of carboxylic acid. A solvent such as 2-butanone was added to the sample prior to analysis to obtain a monophasic system.

Comparative examples were carried out in a similar manner but with fuming hydrochloric acid in an amount to obtain a molar ratio of HCl to carboxylic acid of 0.01.

### Example 1: Preparation of Ethyl n-hexanoate

The reaction vessel was fed with the starting compounds *n*-hexanoic acid, ethanol, HCl and water at a molar ratio of 1 : 1.3 : 0.25 : 0.92 to obtain a mixture of a total weight of 242.5 g. After 8 h reaction time the total yield of ethyl hexanoate was determined to be about 90%.

### Example 2: Preparation of Ethyl n-hexanoate

The reaction vessel was fed with the starting compounds n-hexanoic acid, ethanol, HCl and water at a molar ratio of 1 : 1.3 : 0.1 : 0.37 to obtain a mixture of a total weight of 224.6 g. After 8 h reaction time the total yield of ethyl hexanoate was determined to be about 87%.

### Example 3: Preparation of Ethyl n-hexanoate (Comparative Example)

The reaction vessel was fed with the starting compounds *n*-hexanoic acid, ethanol, HCl and water at a molar ratio of 1 : 1.3 : 0.01 : 0.04 to obtain a mixture of a total weight of 213.8 g. No phase significant separation could be detected. After 8 h reaction time the total yield of ethyl hexanoate was determined to be about 76.2%.

### Example 4: Preparation of Ethyl 3-chloropropionate

The reaction vessel was fed with the starting compounds 3-chloropropionic acid, ethanol, HCl and water at a molar ratio of 1 : 1.3 : 0.25 : 0.92 to obtain a mixture of a total weight of 267.3 g. After 8 h reaction time the conversion of propionic acic was about complete. The total yield of ethyl 3-chloropropionate was determined to be >99%.

### Example 5: Preparation of Ethyl 3-chloropropionate

The reaction vessel was fed with the starting compounds 3-chloropropionic acid, ethanol, HCl and water at a molar ratio of 1 : 1.3 : 0.1 : 0.37 to obtain a mixture of a total weight of 246.9 g. After 8 h reaction time the conversion of propionic acic was about complete. The total yield of ethyl 3-chloropropionate was determined to be 96.5%.

### Example 6: Preparation of Ethyl 3-chloropropionate (Comparative Example)

The reaction vessel was fed with the starting compounds 3-chloropropionic acid, ethanol, HCl and water at a molar ratio of 1 : 1.3 : 0.01 : 0.04 to obtain a mixture of a total weight of 234.5 g. No phase significant separation could be detected. After 8 h reaction time the total yield of ethyl 3-chloropropionate was determined to be 86%.

### Example 7: Preparation of Ethyl chloroacetate

Chloroacetic acid (27.86 g, 0.295 mol, 1 eq.) was reacted with ethanol (23.8 g, 0.517 mol, 1.94 eq.) at 50 °C for 3.5 h in the presence of water (5.42 g, 0.301 mol, 1.13 eq.) and HCl (3.18 g, 0.087 mol, 0.33 eq.). The yield of ethyl chloroacetate was determined to be about 80%.

### Example 8: Preparation of Isopropyl chloroacetate

Chloroacetic acid (25.17 g, 0.266 mol, 1 eq.) was reacted with isopropyl alcohol (31.1 g, 0.517 mol, 1.94 eq.) at 50 °C for 3.5 h in the presence of water (5.42 g, 0.301 mol, 1.13 eq.) and HCl (3.18 g, 0.087 mol, 0.33 eq.). Yield of isopropyl chloroacetate was about 62%.

### Example 9: Preparation of Ethyl benzoate

The reaction vessel was fed with the starting compounds benzoic acid, ethanol, HCl and water at a molar ratio of 1 : 1.3 : 0.25 : 0.92 to obtain a mixture of a total weight of 273.0 g. After 8 h reaction time the total yield of ethyl benzoate was determined to be 56.8%.

### Example 10: Preparation of Ethyl benzoate

The reaction vessel was fed with the starting compounds benzoic acid, ethanol, HCl and water at a molar ratio of 1 : 1.3 :0.1 :0.37 to obtain a mixture of a total weight of 253.6 g. After 8 h reaction time the total yield of ethyl benzoate was determined to be 51.5%.

### Example 11: Preparation of Ethyl benzoate

The reaction vessel was fed with the starting compounds benzoic acid, ethanol, HCl and water at a molar ratio of 1 : 4 : 0.1 : 0.37 to obtain a mixture of a total weight of 259.9 g. After 8 h reaction time the total yield of ethyl benzoate was determined to be 51.6%.

### Example 12: Preparation of Ethyl benzoate (Comparative example)

The reaction vessel was fed with the starting compounds benzoic acid, ethanol, HCl and water at a molar ratio of 1 : 1.3 : 0.01 : 0.04 respectively, to obtain a mixture of a total weight of 243.8 g. No phase significant separation could be detected. After 8 h reaction time the total yield of ethyl benzoate was determined to be 27.1 %.

Table 1 summarizes the amounts of the educts used as well as the product yields obtained. The experiments in which a phase separation was observed at the beginning of the reaction are indicated. Comparative examples are marked with an asterisk.

**Table 1**

| **Example No.** | **n-Hexanoic acid [eq]** | **Ethanol [eq]** | **HCl [eq]** | **Water [eq]** | **Yield Ester [%]** |
|---|---|---|---|---|---|
| 1 | 1.00 | 1.30 | 0.25 | 0.92 | 90.0 |
| 2 | 1.00 | 1.30 | 0.10 | 0.37 | 87.0 |
| 3* | 1.00 | 1.30 | 0.01 | 0.04 | 76.2 |

| | **3-Chloropropionic acid [eq]** | **Ethanol [eq]** | **HCl [eq]** | **Water [eq]** | **Yield Ester [%]** |
|---|---|---|---|---|---|
| 4 | 1.00 | 1.30 | 0.25 | 0.92 | >99.0 |
| 5 | 1.00 | 1.30 | 0.10 | 0.37 | 96.5 |
| 6* | 1.00 | 1.30 | 0.01 | 0.04 | 91.5 |

| | **Chloroacetic acid [eq]** | **Ethanol [eq]** | **HCl [eq]** | **Water [eq]** | **Yield Ester [%]** |
|---|---|---|---|---|---|
| 7 | 1.00 | 1.94 | 0.33 | 1.13 | 80.0 |

| | **Chloroacetic acid [eq]** | **Isopropanol [eq]** | **HCl [eq]** | **Water [eq]** | **Yield Ester [%]** |
|---|---|---|---|---|---|
| 8 | 1.00 | 1.94 | 0.33 | 1.13 | 62.0 |

| | **Benzoic acid [eq]** | **Ethanol [eq]** | **HCl [eq]** | **Water [eq]** | **Yield Ester [%]** |
|---|---|---|---|---|---|
| 9 | 1.00 | 1.30 | 0.25 | 0.92 | 56.8 |
| 10 | 1.00 | 1.30 | 0.10 | 0.37 | 51.5 |
| 11 | 1.00 | 4.00 | 0.10 | 0.37 | 51.6 |
| 12* | 1.00 | 1.30 | 0.01 | 0.04 | 27.1 |

| | | | | | |
|---|---|---|---|---|---|
| *Comparative example | | | | | |

The results show that the use of HCl in a molar ratio of HCl to carboxylic acid of 0.1 :1 and more in the presence of water results in an increased product yield compared to reactions carried out with conventional catalytic amounts of HCl.

### Example 13: Preparation of ethyl 3-chloroacetate to determine the maximum amount of ethanol suitable to maintain a biphasic reaction system over the reaction period

Experiments showed that when chloroacetic acid and ethanol are reacted in the presence of HCl and water in a molar ratio of 1 : 1.3 : 0.2 : 0.75, phase separation occurs at a temperature of 60 °C. As a molar excess of alcohol with respect to the carboxylic acid favourably shifts the equilibrium to the ester product but a molar excess too high destroys the miscibility gap, it is important to determine the maximum amount of alcohol at which the biphasic system is maintained at the reaction temperature of 60 °C.

In order to determine the maximum amount of alcohol at which the biphasic system is maintained at the reaction temperature, the following experiment was designed.

The reaction vessel was fed with the chloroacetic acid (2.79 g, 30 mmol, 0.2 eq.), ethyl chloroacetate (14.58 g, 119 mmol, 0.8 eq.) ethanol (3.4 g, 75 mmol, 0.5 eq.), HCl (36% by weight, 3.0 g, 30 mmol, 0.2 eq. (calculated on 100% HCl), this fuming HCl comprising water (1.98 g, 110 mmol, 0.75 eq) and additional water (2.14 g, 119 mmol, 0.82 eq.). This corresponds to a typical product mixture obtained when starting from a reaction mixture consisting of chloroacetic acid (14.0 g, 148 mmol, 1 eq.), ethanol (8.78 g, 193 mmol, 1.3 eq.), HCl (1.08 g, 30 mmol, 0.2 eq.) and water (1.98 g, 110 mmol, 0.75 eq.) and obtaining the ester in a yield of 80%.

The components fed into the reaction vessel were mixed and heated to a temperature of 60 °C. Ethanol was slowly added to this mixture at 60 °C. Following the addition of 2 g of ethanol (43 mmol, 0.3 eq.), the phase boundaries disappeared. Phase separation only occured again after cooling to room temperature. Thus, for the given starting composition a maximum molar ratio of chloroacetic acid to ethanol of about 1.1.6 is suitable to maintain phase separation at 60 °C.

The maximum molar ratio of chloroacetic acid to ethanol will be lower at higher temperature and will be higher at a higher ratio of water to chloroacetic acid. This allows easy determination of the amounts of water or alcohol at which a biphasic system will form.

## Claims

1. A method for preparing a carboxylic acid ester, wherein a carboxylic acid and/or a salt thereof is reacted with an alcohol in the presence of hydrogen chloride (HCl) and water to form the carboxylic acid ester, wherein the reaction is carried out using a starting reaction system wherein the molar ratio of HCl to carboxylic acid is at least 0.075 : 1 and the molar ratio of water to carboxylic acid is at least 0.25 : 1.

2. The method of Claim 1, wherein the molar ratio of alcohol to carboxylic acid in the starting reaction mixture is in the range of from 0.8 : 1 to 4 : 1, the molar ratio of HCl to carboxylic acid is at least 0.1 : 1, and the molar ratio of water to carboxylic acid is at least 0.35 : 1.

3. The method of Claim 2, wherein the molar ratio of alcohol to carboxylic acid in the starting reaction mixture is in the range of from 1 : 1 to 1.5 : 1, the molar ratio of HCl to carboxylic acid is at least 0.2 : 1, and the molar ratio of water to carboxylic acid is at least 0.7 : 1.

4. The method of any one of claims 1 to 3, wherein the reaction is carried out at a temperature of from 20 to 70 °C.

5. The method of any one of claims 1 to 4, wherein the carboxylic acid is selected from the group consisting of optionally halogenated linear or branched saturated or unsaturated aliphatic C₂₋₂₀ mono- and dicarboxylic acids and optionally halogenated mono- or bicyclic aromatic mono- and dicarboxylic acids.

6. The method of Claim 5, wherein the carboxylic acid is an optionally halogenated linear or branched saturated or unsaturated aliphatic C₂₋₆ mono- or dicarboxylic acid.

7. The method of Claim 6, wherein the carboxylic acid is a saturated chlorinated C₂₋₆ monocarboxylic acid chlorinated with one, two or three chlorine atoms.

8. The method of Claim 7, wherein the saturated chlorinated carboxylic acid is a mono-chlorinated C₂₋₆ monocarboxylic acid.

9. The method of Claim 7 or Claim 8, wherein the saturated chlorinated carboxylic acid is selected from the group consisting of monochloroacetic acid, 2-chloropropionic acid and 3-chloropropionic acid.

10. The method of any one of Claims 7 to 9, further comprising the step of preparing the chlorinated carboxylic acid to be used in the esterification reaction by reacting the corresponding non-chlorinated carboxylic acid with chlorine to form a mixture containing said chlorinated carboxylic acid and HCl, followed by the addition of alcohol, and, optionally, water and HCl to said mixture to obtain the starting reaction system as defined above comprising said chlorinated carboxylic acid.

11. The method of claim 5, wherein the carboxylic acid is a linear or branched saturated or unsaturated aliphatic C₁₂₋₂₀ monocarboxylic acid.

12. The method of claim 5, wherein the carboxylic acid is an optionally halogenated monocyclic aromatic mono- or dicarboxylic acid.

13. The method of any one of Claims 1 to 12, wherein the alcohol is a linear or branched aliphatic C₁₋₈ alcohol, preferably a C₁₋₄ alcohol.

14. The method of Claim 13, wherein the alcohol is ethanol.
